# EUROPEAN PATENT SPECIFICATION

(11) **EP 3 265 181 B1**
(45) Date of publication and mention of the grant of the patent: **10.06.2020**
(21) Application number: 16704564.0
(22) Date of filing: 09.02.2016
(51) Int. Cl.: A61Q 5/00, A61K 8/99, C11D 3/00, C11D 1/36

(54) **COMPOSITIONS WITH REDUCED DYE-TRANSFER PROPERTIES**
ZUSAMMENSETZUNGEN MIT VERRINGERTEN FARBSTOFFÜBERTRAGUNGSEIGENSCHAFTEN
COMPOSITIONS PRÉSENTANT DES PROPRIÉTÉS DE TRANSFERT DE COULEURS RÉDUITES

(30) Priority: 02.03.2015 WO PCT/EP2015/157244
(43) Date of publication of application: 10.01.2018
(73) Proprietor: Unilever PLC, London, Greater London EC4Y 0DY (GB); Unilever NV, 3013 AL Rotterdam (NL)
(72) Inventor: PETKOV, Jordan, Todorov, 47800 Petaling Jaya Selangor (MY); STEVENSON, Paul, Simon, Wirral Merseyside CH63 3JW (GB)
(74) Representative: McHugh, Paul Edward
(86) International application number: PCT/EP2016/052680
(87) International publication number: WO 2016/139032

(56) References cited:
- EP-A1- 0 975 718
- EP-A1- 2 410 039
- WO-A1-2011/120776
- WO-A1-2012/010406
- WO-A1-2013/043857
- WO-A1-2014/095367
- US-A1- 2014 086 864

## Description

This invention relates to compositions for use in aqueous based treatments of coloured or dyed substrates such as fabrics and dishes, with reduced dye transfer.

In certain washing situations dye-loss or dye-fade is undesirable such as the washing of bright/dark fabrics or dyed hair in which wanted dyes are transferred to the washing solution and e.g. in fabric washing, from the substrate to other substrates.

WO 2013/043857 discloses detergent compositions comprising sustainable surfactant systems comprising isoprenoid-derived surfactants.

WO 2014/095367 A1 discloses a personal care composition comprising a) a surfactant comprising (i) a synthetic anionic surfactant; and (ii) a biosurfactant comprising a glycolipid wherein the biosurfactant is present at a level of at least 50 wt.% of the total surfactant combination; b) a fatty acyl isethionate product at a level of 4-10 wt.% of the total personal care composition.

WO 2011/120776 A1 discloses a mild to the skin foaming detergent composition comprising a) 1 to 20 wt.% sophorolipid biosurfactant, b) 1 to 20 wt.% anionic surfactant selected from the group consisting of glycinate, sulphosuccinate, and mixtures thereof, and water.

EP 2 410 039 A1 discloses a cleaning composition comprising mono-rhamnolipid and di-rhamnolipid, characterised in that the ratio of the total wt.% of mono-rhamnolipid to the total wt.% of di-rhamnolipid lies in the range of 95:5 to 45:55.

WO 98/38270 A1 discloses a detergent composition comprising an alkali component, and a dye transfer inhibition additive selected from (i) a sugar surfactant, (ii) an alkyl sulfate, and (iii) an aminocarboxylic acid amphoteric surfactant.

An object of the invention is to provide a composition and a process for personal bathing and hand washing of dishes and/or fabrics with reduced dye-transfer.

According to a first aspect of the present invention there is provided a method of protecting a coloured or dyed substrate from dye transfer during exposure to an aqueous cleansing solution, the method comprising the step of treating a dyed or coloured substrate with a composition comprising a surfactant system, wherein said surfactant system comprises a glycolipid biosurfactant in the range 50-100 wt.% (of the total surfactant system).

Preferably, the surfactant system constitutes the sole surfactant content of the composition.

According to a further aspect of the present invention there is provided use of a washing composition comprising a glycolipid biosurfactant to reduce dye transfer from a coloured or dyed substrate during a washing process.

The substrate is preferably a fabric or hard surface or hair which is coloured or dyed. The colour/dye may be natural or may result from artificial colouring with dyes or pigments or combinations thereof.

Preferably the glycolipid biosurfactant comprises a rhamnolipid.

Preferably the glycolipid biosurfactant comprises a sophorolipid. If sophorolipids are included, acidic forms of sophorolipids are preferred.

The glycolipid biosurfactant may comprise combinations of different glycolipid biosurfactants.

In the case of rhamnolipids, throughout this patent specification, the prefixes mono- and di- are used to indicate respectively to indicate mono-rhamnolipids (having a single rhamnose sugar ring) and di-rhamnolipids (having two rhamnose sugar rings) respectively. If abbreviations are used R1 is mono-rhamnolipid and R2 is di-rhamnolipid. Preferably the ratio of R1:R2 is such that R2 is always greater in proportion to R1, and more preferably the rhamnolipid is 100wt.% R2.

Preferably the glycolipid is present at 75 - 95 wt.% of the surfactant combination.

Most preferably the glycolipid biosurfactant is a rhamnolipid present at 75-95 wt.%

The surfactant combination preferably comprises a synthetic anionic surfactant. 'Anionic surfactants' are defined herein as amphiphilic molecules comprising one or more functional groups that exhibit a net anionic charge when in aqueous solution at the normal wash pH of between 4 and 11.

Preferably the alkali metal salts of organic sulphur reaction products having in their molecular structure an alkyl moiety containing from about 6 to 24 carbon atoms, more greater than 12 carbon atoms and preferably also a moiety selected from the group consisting of sulphonic and sulphuric acid ester moieties. Additionally or alternatively, the anionic surfactant preferably has low levels of ethoxylation, preferably comprising 1-12 ethylene oxide units per molecule, more preferably 1-3 and even more preferably 1. The units of ethylene oxide may be an average.

Providing the formulation scientist with the freedom to use longer carbon chain lengths and/or lower levels of ethoxylation is greatly beneficial, not least on cost grounds. However these factors increase calcium intolerance and so such surfactants are advantageous selections for the present invention.

Although any anionic surfactant hereinafter described can be used, such as primary alkyl sulphates (PAS) e.g. sodium lauryl sulphate (SLS) and e.g. alkyl ether sulphate such as sodium lauryl ether sulphate(SLES), soaps, fatty acid ester sulphonates, fatty acid sulphates or sulphonates; alkyl benzene sulphonates (LAS), sulphosuccinate esters, olefin sulphonates, paraffin sulphonates and organic phosphates; fatty alcohol sulphates; alkyl phenol ether sulphate; fatty acyl isethionate products which products comprise fatty acyl isethionate and free fatty acid and/or fatty acid salt; alkyl sulphonates such as sodium alkane sulphonate. Preferred anionic surfactants are the alkali (ammonium or triethylammonium for example) and alkaline earth metal salts of the above. The source oil/alcohol can be plant or animal derived for example coconut or palm or tallow etc.

The surfactant system is present in the fabric or hard surface washing compositions at a level of from 3 to 85% by weight, preferably from 3 to 60% by weight, more preferably from 3 to 40% by weight, most preferably from 3 to 35% by weight.

The surfactant system is present in personal (human skin and hair) wash compositions at a level of 5 to 60%, preferably 10 to 40% surfactant, while cosmetic compositions need not comprise any surfactant, but preferably comprise 1% to 30% by wt., more preferably 1 to 15% by wt. surfactant.

The compositions of the invention may comprise other ingredients as described hereinbelow.

Hand washing and fabric cleaning compositions may comprise polyester substantive soil release polymers, hydrotropes, opacifiers, colorants, other enzymes, further surfactants such as non-ionic, cationic and or amphoteric surfactants, microcapsules of ingredients such as perfume or care additives, softeners, polymers for anti re-deposition of soil, bleach, bleach activators and bleach catalysts, antioxidants, pH control agents and buffers, thickeners, external structurants for rheology modification, visual cues, either with or without functional ingredients embedded therein and other ingredients known to those skilled in the art.

The compositions of the invention comprise pourable liquids and preferably have a viscosity in the range 250 to 100,000 mPas (cP) measured at a shear rate 10s@-1 and 25 DEG C., in a Haake Rotoviscometer RV20.

Shampoo compositions are preferably in the range from 5000 to 8000 Cp.

Compositions of the invention may be formulated as products for washing fabrics, skin or hair and may include rinse-off, wipe-off and leave-on care products.

The composition is preferably a liquid, gel but may also be a free flowing particulate, paste, or tabletted.

The invention will be further described with reference to the following non-limiting examples.

### Examples Laundry Formulation

*** Denotes a reference example not according to the invention Examples 1* and 2**

| OOA | Name | SUPPLIER | EG 1* as 100% | EG 2 as 100% |
|---|---|---|---|---|
| | | | (%) | (%) |
| 1 | Demin water | Demin water | 27.28 | 27.28 |
| 2 | Tinopal CBS-SP | Ciba | 0.25 | 0.25 |
| 3 | Mono Propylene Glycol MPG | Dow | 8 | 8 |
| 4 | Neodol 25_7 | Shell | 5.04 | 0 |
| 5 | Acusol 820 | Dow | 1 | 1 |
| 6 | Mono ethanol amine | Dow | 6.2 | 6.2 |
| 7 | EU LAS acid - Petresa HF | Petresa HF plus PSU Sul phonation | 6.72 | 0 |
| 8 | Rhamnolipid - JBR425 - Jeneil | Jeneil | 11.2 | 28 |
| 9 | Tri ethanol amine | Dow | 4.035 | 4.035 |
| 10 | Citric Acid | Tate and Lyle | 2.5 | 2.5 |
| 11 | Prifac 5908 | Palmera B1231 - | 3.5 | 3.5 |
| 12 | Dequest 2010 | ex Thermophos | 1.5 | 1.5 |
| 13 | Sodium Sulphite - | Aldrich | 0.25 | 0.25 |
| 14 | EU SLES 3EO - | PSU Sulphonation (synthetic source) | 5.04 | 0 |
| 15 | Sokolan HP20 | BASF | 2 | 2 |
| 16 | Perfume | IFF | 1.39 | 1.39 |
| 17 | Acusol OP301 | Acusol OP301 Dow ○ | 0.1 | 0.1 |

| | | | | |
|---|---|---|---|---|
| Notes For Examples 1 and 2: OOA is the order of addition upon making the formulation Tinopal CBS SP Slurry 33 a Distyryl biphenyl derivative CAS No. 27344-41-8 Acusol 820, a copolymer of acrylic acid with C18 and with EO20C18 side chains, MW about 500,000 Prifac 5908 is Hydrogenated Topped Palm Kernel Fatty Acids = Dequest 2010 is 1-Hydroxy) ethylidene -1,1,-diphosphonic acid, HEDP Sokolan HP20 is ethoxylated polyethylene imine Neodol 25-7 is is a primary C12 - C15 Alcohol Ethoxylate with average of 7 moles of ethylene oxide per mole of alcohol Acusol 820 is Hydrophobically modified Alkali soluble acrylic polymer emulsion EU LAS is Linear Alkyl Benzene Sulphonate SLES 3EO is Sodium Lauryl Ether Sulphate with average distribution of 3 moles of ethylene oxide per mole of Sodium Lauryl Sulphate Rhamnolipid JBR 425 is A mixture of mono and Di rhamnolipids where the IUPAC name for mono rhamnolipid is 3-13-[(2R,3R,4R,5R,6S)-3,4,5-trihydroxy-6-methyloxan-2-yl]oxydecanoyloxy]decanoic acid and Di rhamnolipid is - 3-[3-[4,5-dihydroxy-6-methyl-3-(3,4,5-trihydroxy-6-methyloxan-2-yl)oxyoxan-2-yl]oxydecanoyloxy]decanoic acid Soladona 2012 is perfume supplied by IFF Accusol OP301 = Is an opacifier supplied as an emulsion | | | | |

**Examples 3-6* Hair Compositions**

| **INCI name** | **Tradename** | **Ex 3* wt.%** | **Ex 4* wt.%** | **Ex 5* wt.%** | **Ex 6* wt.%** |
|---|---|---|---|---|---|
| ethoxylated alkyl sulfate anionic surfactant having an average degree of ethoxylation of from 0.5 to 3, preferably 1 to 3 and most preferably 1. | Texapon N701 | 4.0 | 10.0 | 10.0 | 6.0 |
| BIOSURFACTANT ⁴ | | 4.0 | 5.0 | 5.0 | 3.0 |
| Cocamidopropyl Betaine | Tegobetaine CK | 3.0 | 3.0 | 1.6 | 3.0 |
| Fatty Acyl Isethionate Product¹ (DEFI) | Unilever | 7.0 | 2.0 | 4.0 | 3.0 |
| Sodium Cocoyl Isethionate | Jordapon Cl | - | - | - | 3.0 |
| Carbomer | Carbopol 980 | 0.60 | 0.60 | 0.60 | 0.60 |
| Silicone Oil² | | 2.2 | 2.2 | 2.2 | 2.2 |
| Guar Hydroxypropyltrimonium Chloride | BFG-Jaguar C17 | 0.25 | 0.25 | 0.25 | 0.25 |
| Parfum | Snow White - Givaudan | 0.75 | 0.75 | 0.75 | 0.75 |
| Aqua + minors | Water + minors | to 100 | to 100 | to 100 | to 100 |

| | | | | | |
|---|---|---|---|---|---|
| ¹ The Fatty Acyl Isethionate product is Sodium Cocoyl isethionate, Stearic Acid, Coconut Fatty Acid, Sodium Isethionate and Water produced in-house by Unilever ² Mixture of silicone emulsions from Wacker and Dow ³ The Viscosity of the formulations was measured using a Brookfield viscometer at 30°C and 20rpm using spindle N5; all were in the range of from 5000 to 8000 Cp ⁴The biosurfactant is Rhamnolipid JBR425 (CAS no. 147858-26-2) ex. Jeneil Biosurfactant Co., LLC | | | | | |

### Experimental

The following experiment was carried out to assess the removal of hair dye from dyed hair swatches by different surfactant systems in solution

### Method

The method measures absorbance, at a wavelength that corresponds to the concentration of dye in solution, of a supernatant solution. The supernatant solution is produced after soaking dyed hair swatches in various detergent solutions that are made up either with or without the presence of Biosurfactants in a controlled test. The lower the absorbance the less amount of dye remains in the supernatant solution and consequently is indicative of less removal of dye from the hair swatch.

### Materials

- SLES - 1EO - Texapon N71 - Shell
- JBR425 - Rhamnolipid - Jeneil
- Permanent Hair colourant - having a shade level 1 (black) or 2 (darkest brown) according to the International Colour Chart (ICC).

### Colour loss method

- 2.5g 6" hair switches previously bleached * were dyed using a commercially available permanent colourant
- 10g of the colourant were applied to the hair switch and rubbed through for 30 seconds using a brush until colour is evenly distributed.
- The switch was then left for 35 minutes before rinsing for 2 minutes under controlled temperature (37°C) and flow (4L/min).
- 150g of the bio-surfactant and/or synthetic Surfactant solutions or combination of ( as outlined in the results section under the title surfactant) were prepared at 1% w/w and the switch was then placed into the surfactant solution and left for 1 hour at ambient temperature.
- After 1 hour, the switch was removed from the surfactant. A sample of the resulting solution was then added to a cuvette and the absorbance measured with a Jasco 600 UV spectrometer at a set wavelength of 522nm.

### * Preparation of Switches - Bleaching

### Equipment required:

- L'Oreal Platine Precision Powder Bleach
- Excel Crème Peroxide - 9% Vol
- Tinting Bowl
- Tinting Brush
- Aluminium Foil
- Stop clock

All rinsing and washing to be done using the flow/temperature controlled taps. The flow rate is set at 4 litres / minute and a temperature of 35°C - 40°C.

| Number of Switches | Type of Switch | Amount of | Amount |
|---|---|---|---|
| | | Bleach Powder (g) | of Peroxide (9) |
| 4 | 10" | 60 | 120 |
| 5 | 6" | 30 | 60 |

### First bleach application...

1. Lay out four sheets of Aluminium Foil on the bench large *enough*
   *to allow for the switch to be wrapped in the foil to develop once the bleach has been applied.*
2. Weigh out the bleach powder into a tinting bowl.
3. Weigh out the creme peroxide by gently pouring it over the powder so that the powder is completely submerged. Mix into a creamy consistency Using an IKA-WERKE Eurostar Power Control-Visc Overhead Stirrer with a whisk attached. Mix for 60 seconds at 400 rpm. The tinting bowl must be held firmly flat in place. Ensure that there are no lumps as this will give an uneven colour on the switches. Once the mixture is prepared it must be used immediately.
4. Spread one of the switches (in a fan shape) on the sheet of foil.
5. Apply the bleach mixture with a tinting brush. Ensure even coverage of the switch by turning it and applying the bleach to each side twice.
   - Side 1 apply for 45 seconds, turn over
   - Side 2 apply for 45 seconds, turn over
   - Side 1 apply for 45 seconds, turn over
   - Side 2 apply for 45 seconds
6. Bring the hair fibres together and leave the switch in its normal shape, wrap the switch in the aluminium foil and leave to develop at ambient temperature for **30 minutes**. Note the time that the switch needs rinsing on the top of the aluminium foil using a permanent marker.
7. Repeat with the rest of the switches.
8. When the developing time is finished remove the switch from the foil and rinse for 2 minutes under the tap, running the fingers down the switch every 20 seconds.
   **The hair must be rinsed completely. If any bleach is left in, it will continue to develop.**
9. Lay the switch down on the edge of the sink and using the WIDE teeth of a Matador Sawcut No4 comb; carefully comb the tangles out of the switch. *Start at the tip end and work up slowly to the root.* Once all the tangles have been combed out finish with the NARROW teeth of the comb.
10. Run the first and middle finger down the switch and dry at an ambient temperature overnight.

**Second bleach application... Repeat steps 1-10 above and once completed wash and rinse the hair swatches completely to remove any residual bleach.**

### Results

| Surfactant | Total Conc (w/w%) | pH | Soak Time | Soak Temp | Hair Type |
|---|---|---|---|---|---|
| SLES - Texapon N71 | 1 | 7 | 1Hr | 37 Deg C | 2 x Bleached then Coloured Black |
| JBR425 - Jeneil | 1 | 7 | 1Hr | 37 Deg C | 2 x Bleached then Coloured Black |
| SLES/JBR425 (50:50 mix) | 1 | 7 | 1Hr | 37 Deg C | 2 x Bleached then Coloured Black |

| **Surfactant mix** | **Absorbance at 522nm** |
|---|---|
| SLES- Texapon N71 | 3.9463 |
| JBR425 | 3.0079 |
| SLES/JBR425 50:50 mix | 3.651 |

The data shows that the amount of dye released into solution, from the dyed hair swatch, at the end of the process, is significantly greater for the SLES based surfactant system. Replacing the SLES with rhamnolipid significantly reduces the amount of dye loss from hair during the hair wash process with the rhamnolipid, and consequently increases the lifetime of a hair dye.

## Claims

1. A method of protecting a coloured or dyed substrate from dye transfer during exposure to an aqueous cleansing solution, the method comprising the step of treating said a dyed or coloured substrate with a composition comprising a surfactant system, wherein said surfactant system comprises a glycolipid biosurfactant in the range 50-100 wt.% of the total surfactant system.

2. A method according to claim 1 wherein the method further comprises the step of applying a dye to the substrate.

3. A method according to claim 1 or claim 2 wherein the method comprises the step of applying an aqueous solution comprising the composition to the substrate for a time period of 1 minute to 2 hours before rinsing.

4. A method of dyeing a substrate, the method comprising the steps of
a. Applying a dye to the substrate and,
b. Applying to the substrate a composition comprising a surfactant system comprising 50-100 wt.% glycolipid biosurfactant with respect to of the total surfactant system.

5. A method according to claim 4 wherein step b. follows step a.

6. A method according to any of claims 3-5 comprising the further step of rinsing the applied dye and/or glycolipid biosurfactant from the substrate using water.

7. A method according to any preceding claim wherein the substrate is a fabric and/or human hair.

8. A method according to any preceding claim wherein the glycolipid biosurfactant comprises a rhamnolipid or a sophorolipid.

9. A method according to claim 8 wherein the rhamnolipid comprises a ratio of mono-rhamnolipid: di-rhamnolipid (R1:R2) such that R2 is always greater in proportion by weight to R1.

10. A method according to claim 9 wherein the rhamnolipid is 100wt.% di-rhamnolipid (R2).

11. Use of a washing composition comprising a glycolipid biosurfactant to reduce dye transfer from a coloured or dyed substrate during a washing process.

12. Use according to claim 11 wherein the substrate is human hair and/or fabric.

13. A kit for dyeing hair comprising a dye composition and a composition comprising a glycolipid biosurfactant.

## Patentansprüche

1. Verfahren zum Schutz eines farbigen oder gefärbten Substrats vor der Farbübertragung, während es einer wässrigen Reinigungslösung ausgesetzt ist, wobei das Verfahren den Schritt des Behandelns des gefärbten oder farbigen Substrats mit einer Zusammensetzung umfasst, die ein Tensidsystem umfasst, wobei das Tensidsystem ein Glycolipid-Biotensid in dem Bereich von 50-100 Gew.-% des gesamten Tensidsystems umfasst.

2. Verfahren nach Anspruch 1, wobei das Verfahren ferner den Schritt des Auftragens eines Farbstoffs auf das Substrat umfasst.

3. Verfahren nach Anspruch 1 oder Anspruch 2, wobei das Verfahren den Schritt des Auftragens einer wässrigen Lösung, umfassend die Zusammensetzung, auf das Substrat für eine Zeitdauer von 1 Minute bis 2 Stunden vor dem Spülen umfasst.

4. Verfahren zum Färben eines Substrats, wobei das Verfahren die Schritte umfasst:
a. Auftragen eines Farbstoffs auf das Substrat und
b. Auftragen einer Zusammensetzung auf das Substrat, die ein Tensidsystem umfasst, umfassend 50-100 Gew.-% Glycolipid-Biotensid bezüglich des gesamten Tensidsystems.

5. Verfahren nach Anspruch 4, wobei der Schritt b. dem Schritt a. folgt.

6. Verfahren nach irgendeinem der Ansprüche 3-5, umfassend den weiteren Schritt des Abspülens des aufgetragenen Farbstoffs und/oder des Glycolipid-Biotensids von dem Substrat unter Verwendung von Wasser.

7. Verfahren nach irgendeinem vorhergehenden Anspruch, wobei das Substrat ein Textil und/oder menschliches Haar ist.

8. Verfahren nach irgendeinem vorhergehenden Anspruch, wobei das Glycolipid-Biotensid ein Rhamnolipid oder ein Sophorolipid umfasst.

9. Verfahren nach Anspruch 8, wobei das Rhamnolipid ein Verhältnis von Mono-rhamnolipid: Di-rhamnolipid (R1:R2) derartig umfasst, dass R2 im Gewichtsverhältnis zu R1 stets größer ist.

10. Verfahren nach Anspruch 9, wobei das Rhamnolipid 100 Gew.-% Di-rhamnolipid (R2) darstellt.

11. Verwendung einer Waschzusammensetzung, umfassend ein Glycolipid-Biotensid, um die Farbübertragung von einem farbigen oder gefärbten Substrat während eines Waschvorgangs zu verringern.

12. Verwendung nach Anspruch 11, wobei das Substrat menschliches Haar und/oder ein Textil ist.

13. Kit zum Färben von Haar, umfassend eine Farbstoffzusammensetzung und eine Zusammensetzung, umfassend ein Glycolipid-Biotensid.

## Revendications

1. Procédé de protection d'un substrat coloré ou teint à partir de transfert de colorant pendant une exposition à une solution nettoyante aqueuse, le procédé comprenant l'étape de traitement dudit un substrat teint ou coloré avec une composition comprenant un système de tensioactif, dans lequel ledit système de tensioactif comprend un biotensioactif de glycolipide dans l'intervalle de 50-100 % en masse du système de tensioactif total.

2. Procédé selon la revendication 1, dans lequel le procédé comprend de plus l'étape d'application d'un colorant au substrat.

3. Procédé selon la revendication 1 ou la revendication 2, dans lequel le procédé comprend l'étape d'application d'une solution aqueuse comprenant la composition au substrat sur une période de temps de 1 minute à 2 heures avant rinçage.

4. Procédé de coloration d'un substrat, le procédé comprenant les étapes de
a. application d'un colorant au substrat et,
b. application au substrat d'une composition comprenant un système de tensioactif comprenant 50-100 % en masse de biotensioactif de glycolipide par rapport au système de tensioactif total.

5. Procédé selon la revendication 4, dans lequel l'étape b. suit l'étape a.

6. Procédé selon l'une quelconque des revendications 3-5 comprenant l'étape supplémentaire de rinçage des colorant et/ou biotensioactif de glycolipide appliqués du substrat en utilisant de l'eau.

7. Procédé selon l'une quelconque des revendications précédentes, dans lequel le substrat est un textile ou des cheveux humains.

8. Procédé selon l'une quelconque des revendications précédentes, dans lequel le biotensioactif de glycolipide comprend un rhamnolipide ou un sophorolipide.

9. Procédé selon la revendication 8, dans lequel le rhamnolipide comprend un rapport de mono-rhamnolipide : di-rhamnolipide (R1:R2) de sorte que R2 est toujours supérieur en proportion en masse à R1.

10. Procédé selon la revendication 9, dans lequel le rhamnolipide est 100 % en masse de di-rhamnolipide (R2).

11. Utilisation d'une composition de lavage comprenant un biotensioactif de glycolipide pour réduire le transfert de colorant d'un substrat coloré ou teint pendant un procédé de lavage.

12. Utilisation selon la revendication 11, dans laquelle le substrat est des cheveux humains et/ou un textile.

13. Kit pour coloration de cheveux comprenant une composition de colorant et une composition comprenant un biotensioactif de glycolipide.
